# EUROPÄISCHE PATENTSCHRIFT

(11) **EP 1 527 743 B1**
(45) Veröffentlichungstag und Bekanntmachung des Hinweises auf die Patenterteilung: **29.07.2009**
(21) Anmeldenummer: 04090402.1
(22) Anmeldetag: 21.10.2004
(51) Int. Cl.: A61B 18/12, A61B 18/14, A61B 17/30

(54) **Medizingerät für die Elektrotomie**
Medical device for electrotomy
Dispositif médical pour électrotomie

(30) Priorität: 29.10.2003 DE 10351818; 16.12.2003 DE 10361142
(43) Veröffentlichungstag der Anmeldung: 04.05.2005
(73) Patentinhaber: Celon AG Medical Instruments, 14513 Teltow (DE)
(72) Erfinder: Desinger, Kai, 14195 Berlin (DE); Stein, Thomas, 14513 Teltow (DE); Roggan, André, 12163 Berlin (DE)
(74) Vertreter: Eisenführ, Speiser & Partner

(56) Entgegenhaltungen:
- WO-A-02/45589
- WO-A-94/20025
- WO-A-20/04045436
- DE-A1- 3 447 156
- DE-A1- 3 623 340
- DE-A1- 19 957 919
- DE-C1- 10 042 096
- GB-A- 2 154 881
- JP-A- 2001 029 353
- US-A- 5 562 503
- US-A- 5 633 578
- US-A- 5 766 165
- US-A- 6 113 596
- US-B1- 6 190 385

## Beschreibung

Die Erfindung betrifft eine Medizingeräteanordnung für das Trennen oder Entfernen von Körpergewebe mittels Elektrotomie. Die Medizingeräteanordnung umfasst einen Generator für einen hochfrequenten Wechselstrom und ein Schneidinstrument. Die Erfindung betrifft ebenfalls ein Greifinstrument zur Verwendung mit der Medizingeräteanordnung, ein Schneidinstrument für die Medizingeräteanordnung und eine entsprechende Konvertereinheit.

Die Nutzung elektrochirurgischer Verfahren zur Gewebetrennung oder Gewebeentfernung (Elektrotomie) ist bereits seit Jahrzehnten ein Routineverfahren in der Chirurgie. Solche Verfahren bieten den Vorteil, dass das Gewebe in Form eines sogenannten Schmelzschnittes getrennt wird, bei dem von der Schneidelektrode eine Funkenentladung ausgeht, die das Gewebe in unmittelbarer Umgebung der Schneidelektrode zur Verdampfung bringt und kleine Kapillargefäße umgehend verschließt, so dass ein fast blutfreies Gewebeschneiden möglich ist.

Dieses Verfahren wird in allen chirurgischen und anderen medizinischen Disziplinen zur Gewebetrennung oder Gewebeentfernung eingesetzt. Die elektrochirurgische Applikationstechnik wird nach dem Stand der Technik in der sogenannten monopolaren Anwendungstechnik eingesetzt, das heißt, es wird neben der als Aktivelektrode ausgebildeten Schneidelektrode eine großflächige Rückleitelektrode (auch Neutralelektrode genannt) an den Extremitäten des behandelnden Patienten angebracht um einen hochfrequenten Stromfluss zu gewährleisten. Diese monopolare Anwendungstechnik hat den Nachteil, dass der Strom über den gesamten Patienten abfließt. Der Nachteilt resultiert zum einen daraus, dass die monopolare Anwendungstechnik ein immanentes Gefahrenpotential für den Patienten und den Anwender darstellt, da der Strom durch den gesamten Patienten fließt und Verbrennungen durch vagabundierende Leckströme und eine Neutralelektrode nicht ausgeschlossen werden können. Der Nachteil resultiert weiterhin daraus, dass ein Teil der für die Elektrotomie, insbesondere die Funkenentladung benötigte Energie auf seinem Weg zur Neutralelektrode in Wärme umgesetzt wird, so dass der Wirkungsgrad einer entsprechenden Anordnung schlecht ist.

In den letzten Jahren wurden daher einige Konzepte zur elektrochirurgischen Gewebetrennung in bipolarer Applikationstechnik untersucht, die jedoch mit der Qualität eines monopolaren Systems nicht konkurrieren können. Dieses liegt daran, dass bei der Bipolartechnik beide Elektroden in unmittelbarer Nähe im Zielgebiet des Eingriffs nebeneinander angeordnet sein müssen. Da aus physikalischen Gründen jedoch immer nur eine Elektrode, selbst bei gleicher Geometrie, sich zu einer Aktivelektrode ausbilden kann, an der die Funken entstehen, wird die andere Elektrode quasi als Rückleitelektrode mitgeschleppt, was zu einer stark verminderten Schnittqualität führt.

Die japanische Patentanmeldung JP 2001-029353 A offenbart eine elektrochirurgische Einrichtung mit einem Generator, einem Greifinstrument und einem Instrument für Ultraschallchirurgie, die so betrieben werden können, dass zwischen dem Greifinstrument und dem Instrument für Ultraschallchirurgie ein hochfrequenter Wechselstrom fließt.

Der Erfindung liegt daher die Aufgabe zu Grunde, eine Medizingeräteanordnung und Komponenten für das Schneiden von Körpergewebe anzugeben, welche die vorgenannten Nachteile möglichst vermeidet. Ebenso sollen Komponenten für eine derartige Anordnung angegeben werden.

Diese Aufgabe wird erfindungsgemäss durch die Medizingeräteanordnung gemäss Anspruch 1 gelöst, vorteilhafte Ausgestaltungen ergeben sich aus den abhängigen Ansprüchen.

Die Erfindung soll nun anhand von Ausführungsbeispielen näher erläutert werden. Von den Figuren zeigen:
- Figur 1: eine Medizingeräteanordnung
- Figur 2: einen Überblick über eine Vorrichtung zum bipolaren Schneiden von biologischem Gewebe
- Figur 3: einen Schaltplan einer Konvertereinheit für eine Vorrichtung gemäß Figur 1
- Figur 4: eine Medizingeräteanordnung ähnlich Figur 1 mit externer Konvertereinheit
- Figur 5: ein distales Ende eines Greifarmes eines Greifinstrumentes mit aufgestecktem Greifansatz
- Figur 6a bis c: alternative distale Enden der Greifarme eines Greifinstrumentes
- Figur 7: ein alternatives Schneidinstrument in Form einer koagulierenden Schere
- Figur 8: eine beispielhafte Darstellung des Gebrauchs der Vorrichtung aus Figur 2.
- Figur 9: eine alternative Anordnung der Vorrichtung in schematischer Darstellung.

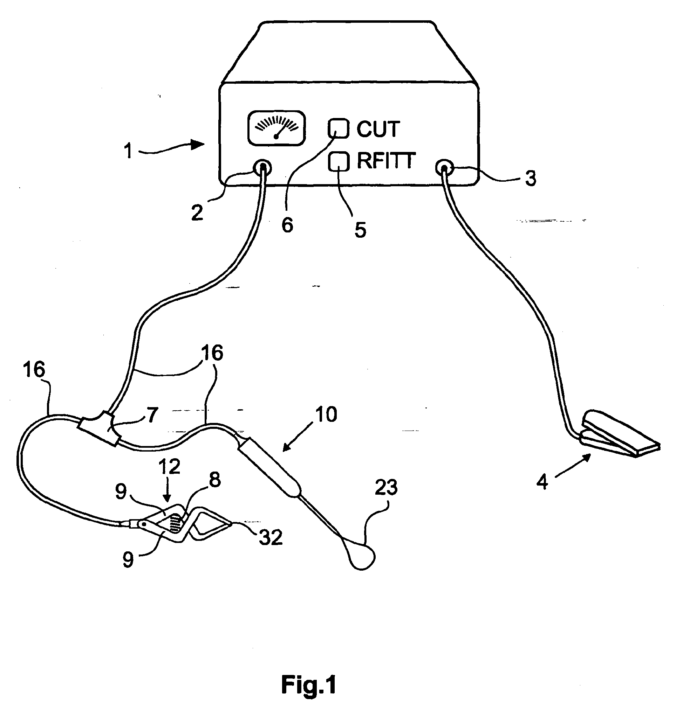

Die Medizingeräteanordnung aus Figur 1 zeigt ein Steuergerät 1 mit integriertem Generator. Das Steuergerät hat einen Steueranschluss 3, mit dem ein Fußschalter 4 verbunden ist. Außerdem hat das Steuergerät 1 einen bipolaren Anschluss 2, an den über elektrische Zuleitungen 16 und eine Leitungsverzweigung 7 einerseits ein Schneidinstrument 10 und andererseits ein Greifinstrument 12 angeschlossen sind, und zwar derart, dass ein hochfrequenter Wechselstrom bipolar zwischen einer Schneidelektrode 23 des Schneidinstrumentes 10 einerseits und Greifoberflächen 32 des Greifinstrumentes 12 als Gegenelektrode andererseits anliegt, sobald der Fußschalter 4 betätigt wird.

Das Steuergerät 1 umfasst einen Generator und Druckschalter 5 und 6, die bewirken, dass je nachdem, welcher der beiden Schalter betätigt ist, am bipolaren Ausgang 2 entweder ein für die interstitielle Thermotherapie (RFITT) benötigte Wechselstrom mit einer spitzen Spitzenamplitude von 80 V anliegt oder ein für das Schneiden geeignete Wechselstrom mit einer spitzen Spitzenamplitude von 200 V.

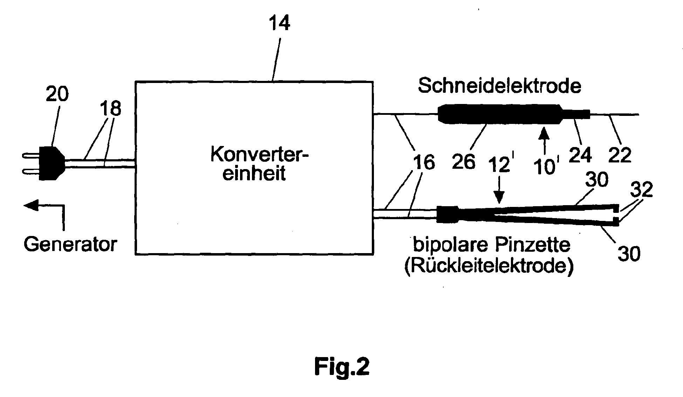

Figur 2 zeigt diejenigen Bestandteile einer Medizingeräteanordnung, die an einen Generator für einen hochfrequenten Wechselstrom anzuschließen sind. Der Generator selbst ist nicht abgebildet.

In Figur 2 dargestellt sind zum einen ein Schneidinstrument 10' und ein als Pinzette ausgebildetes Greifinstrument 12. Außerdem zeigt Figur 2 schematisch eine Konvertereinheit 14, an welche einerseits das Schneidinstrument 10' und andererseits das Greifinstrument 12 über entsprechende elektrische Zuleitungen 16 angeschlossen ist. Die Konvertereinheit 14 weist eingangsseitig ein Kabel 18 mit daran angeschlossenem Anschlussstecker 20 für den Anschluss der Konvertereinheit 14 an einen Generator auf.

Das Schneidinstrument 10' weist eine Schneidelektrode 22 auf, die distal an einem Elektrodenschaft 24 angeordnet ist und über den Elektrodenschaft 24 mit einem Handgriff 26 des Schneidinstrumentes verbunden ist. Schneidelektrode 22 samt Elektrodenschaft 24 sind lösbar mit dem Handgriff 26 verbunden, so dass die Schneidelektrode 22 samt Elektrodenschaft 24 nach einer Operation auszutauschen ist, während der Handgriff 26 wieder zu verwenden ist. Am Handgriff 26 befindet sich ein Anschluss für diejenige elektrische Zuleitung 16, mit der das Schneidinstrument 10 mit der Konvertereinheit 14 verbunden ist. Diese elektrische Verbindung ist lösbar. Außerdem ist im Bereich des Handgriffes 26 ein Druckschalter angeordnet, der mit einem Finger zu bedienen ist und eine elektrische Verbindung zur Schneidelektrode 22 unterbrechen oder wiederherstellen kann.

Alternativ zu einer stabförmigen, angespitzten Schneidelektrode 22 kann auch eine Schneidelektrode in Form einer Drahtschlaufe 23 vorgesehen sein, wie sie in Figur 1 abgebildet ist.

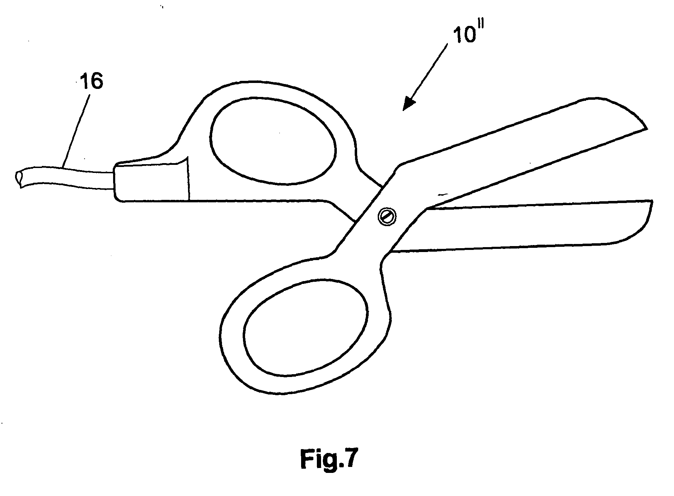

Schließlich zeigt Figur 7 ein alternatives Schneidinstrument in Form einer koagulierenden Schere 10". Diese Schere 10" ist mit über eine elektrische Zuleitung 16 mit dem Steuergerät 1 zu verbinden mit der Folge, dass an die Schneiden der Schere ein elektrisches Potential anzulegen ist. Dies erlaubt es, mit der Schere, wie von üblichen Scheren bekannt, zu schneiden, wobei gleichzeitig auf Grund des anliegenden Potentials im Bereich des Schnittes eine Gewebeverödung eintritt (Koagulation), die eventuell geschnittene Blutgefäße sofort schließt.

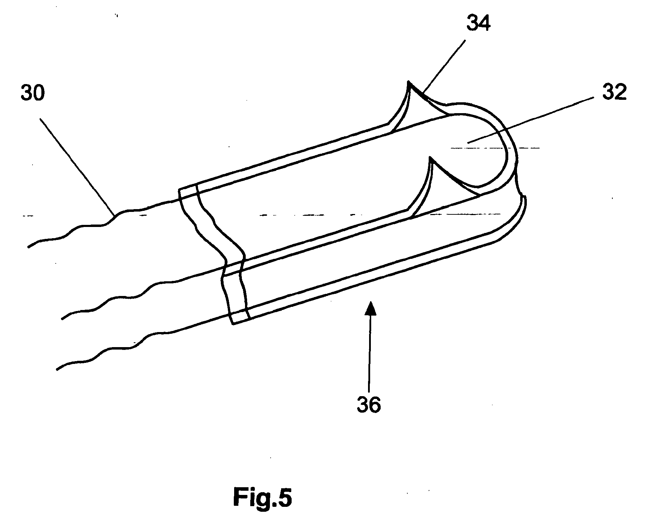

Das Greifinstrument 12' ist entsprechend der eingangs beschriebenen ersten Variante als bipolare Pinzette ausgebildet und daher über zwei elektrische Zuleitungen 16 mit einem Pol der Konvertereinheit 14 und über die Konvertereinheit 14 mit einem Pol des Generators verbunden. Das Greifinstrument 12' weist zwei Greifarme 30 auf, welche am distalen Ende einander zugewandte Greifoberflächen 32 aufweisen. Jede der beiden Greifoberflächen 32 ist mit einer der elektrischen Zuleitungen 16 elektrisch verbunden und wenigstens in Teilbereichen elektrisch leitend ausgebildet. Die Greifoberflächen 32 weisen in Figur 1 nicht erkennbare Erhebungen in Form von Fasszähnen 34 auf, wie sie der Figur 5 zu entnehmen sind. Figur 5 ist weiterhin zu entnehmen, dass eine jeweilige Greifoberfläche 32 samt der Fasszähne 34 Bestandteil eines Greifansatzes 36 sind, die aus Edelstahl besteht und auf das distale Ende eines jeweiligen Greifarmes 32 des Greifinstrumentes 12 aufzuschieben ist. Auf diese Weise können nach einer Operation die entsprechenden Greifansätze ausgewechselt und das übrige Greifinstrument 12 wieder verwendet werden.

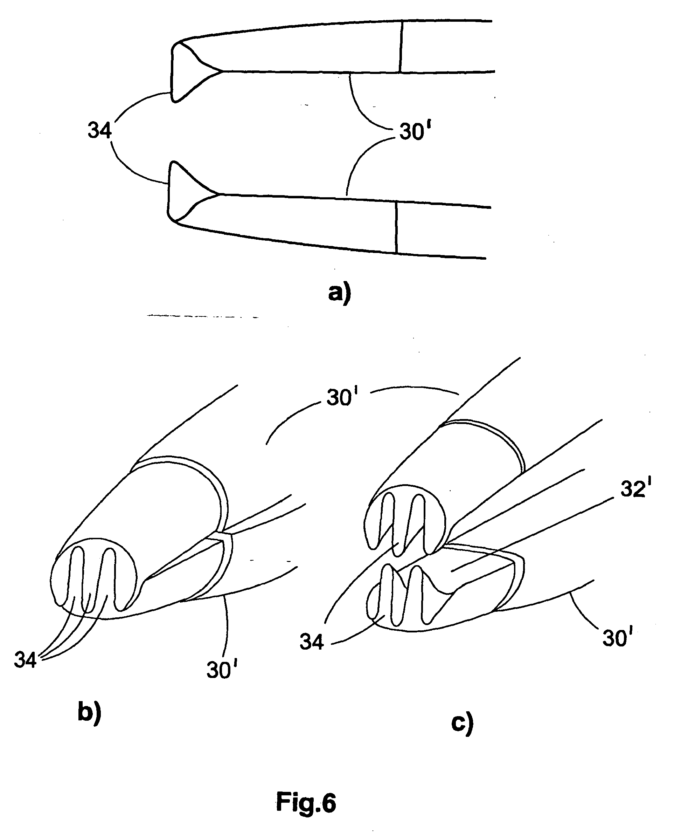

Figur 6 zeigt eine alternative Gestaltung des distalen Endes der Greifarme 30' eines Greifinstrumentes 12 oder 12', wie es in Figuren 1 oder 2 dargestellt ist. Figur 6a zeigt die Greifarme 30' in der Seitenansicht. In Figuren 6b und c ist das distale Ende der Greifarme 30' perspektivisch einmal im geschlossenen und einmal im geöffneten Zustand dargestellt.

In einer alternativen Ausführungsvariante ist das Greifinstrument nicht als herkömmliche Pinzette gestaltet, sondern als selbstschließende Pinzette, wie sie in Figur 1 in Form des Greifinstrumentes 12 schematisch dargestellt ist. Eine Druckfeder 8 führt dazu, dass sich das Greifinstrument 12 selbsttätig schließt und durch Druck auf die Griffflächen 9 gegen die Federkraft der Druckfeder 8 öffnen lässt. Ein derartiges Greifinstrument 12 bietet den Vorteil, dass der behandelnde Arzt mit der das Greifinstrument 12 führenden Hand nicht gleichzeitig ständig die erforderliche Haltekraft ausüben muss.

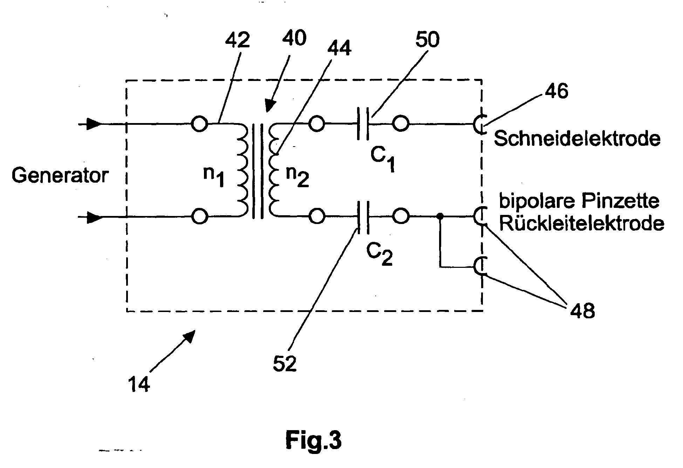

Figur 3 zeigt einen Schaltplan der Konvertereinheit 14, aus dem hervorgeht, dass die Konvertereinheit 14 einen Transformator 40 aufweist, der eine Primärwicklung 42 besitzt, die zum Anschluss an den Generator gedacht ist und eine Sekundärwicklung 44, die einerseits mit einem Anschluss 46 für das Schneidinstrument und andererseits mit zwei parallel geschalteten Anschlüssen 48 für das Greifinstrument verbunden ist. Zwischen der Sekundärwicklung 44 und der Schneidelektrode 46 ist ein erster Kondensator 50 angeordnet. Auf der anderen Seite der Sekundärwicklung ist zwischen der Sekundärwicklung 44 und den Anschlüssen 48 ein zweiter Kondensator 52 angeordnet. Erster und zweiter Kondensator 50 und 52 können zur Impedanzanpassung ausgetauscht werden. Die Kondensatoren haben weiterhin die Wirkung, Gleichstromanteile und damit Faradaysche Effekte zu unterdrücken.

Der Transformator 40 ist im übrigen ein Hochfrequenztransformator, bei dem das Verhältnis der Windungszahl n₂ der Sekundärwicklung 44 zur Windungszahl n₁ der Primärwicklung 42 n₂ > n₁ ist. Ein derartiger Hochfrequenztransformator transformiert eine niedrige, für den Zweck einer Gewebekoagulation ausgelegte Ausgangsspannung eines üblichen Generators (in etwas 80 V) Hochspannungswerte hoch, die das Zünden eines Funkens einer Schneidelektrode und damit das Durchtrennen von biologischem Gewebe ermöglichen. Die Ausgangsspannung an der Konvertereinheit 14 ist beispielsweise etwa 200 V.

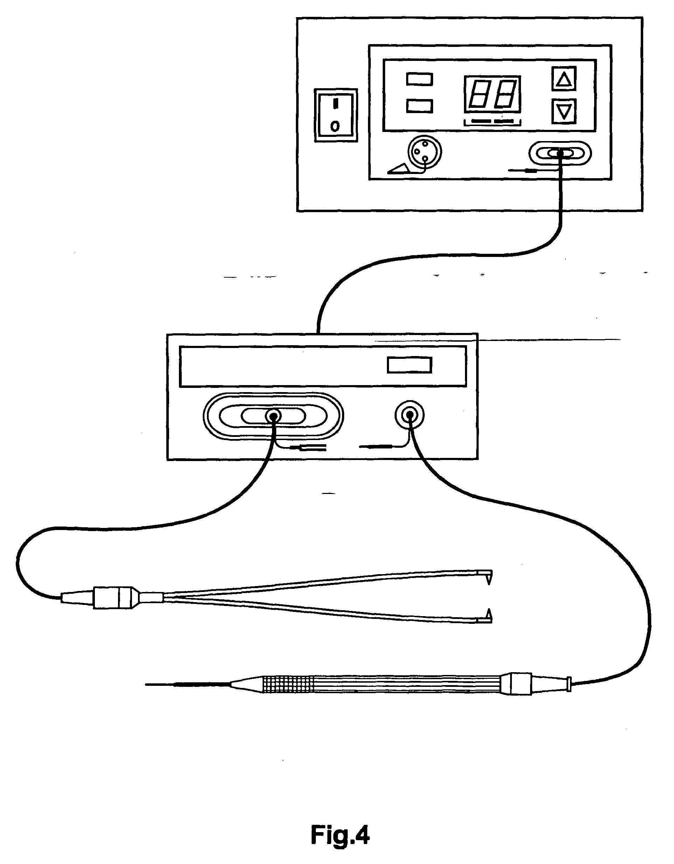

In Figur 4 ist eine Medizingeräteanordnung ähnlich Figur 1 dargestellt, bei der eine Konvertereinheit 14, wie in den Figuren 2 und 3 abgebildet, zwischen Steuergerät 1' einerseits und Greif- sowie Schneidinstrument 12 bzw. 10' andererseits, geschaltet ist. Die Konvertereinheit 14 übernimmt in dieser Anordnung die Funktion der Leitungsverzweigung (7) aus Figur 1. In dem in Figur 4 dargestellten Ausführungsbeispiel ist das Greifinstrument 12 als Pinzette mit Fasszähnen (siehe auch Figur 5) und ist das Schneidinstrument 10' mit angespitzter Schneidelektrode dargestellt. Anstelle dieser Instrumente können aber auch beliebige der hierin dargestellten Schneid- und Greifinstrumente verwendet werden.

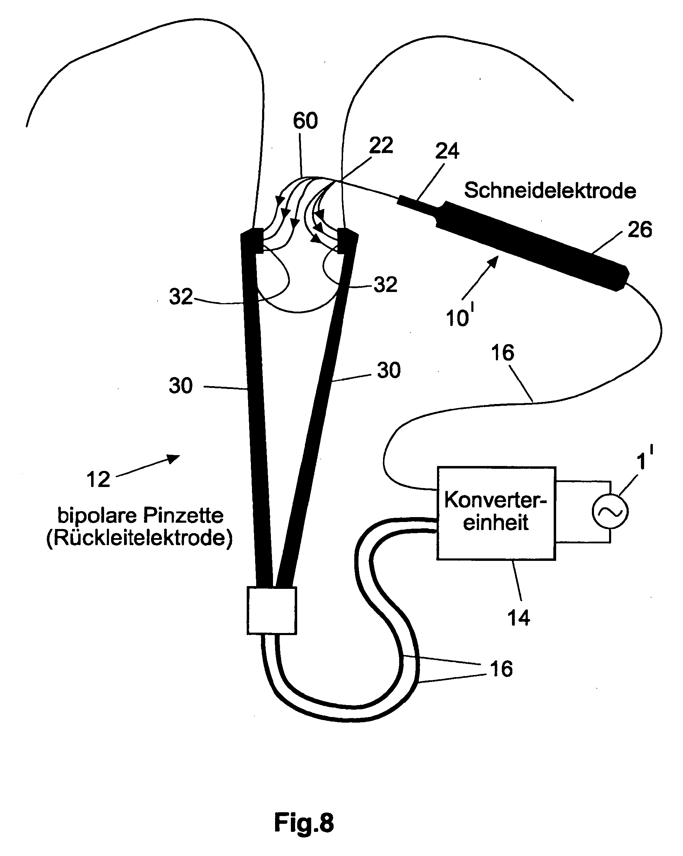

In Figur 8 ist schließlich ein Anwendungsfall für die zuvor beschriebene Medizingeräteanordnung dargestellt, und zwar konkret am Beispiel des Kürzens einer Uvula. Mit den Greifoberflächen 32 greift das Greifinstrument 12 die Uvula. Das Greifinstrument 12 ist, wie in Figur 2 dargestellt, quasi monopolar an die Konvertereinheit 14 angeschlossen und bildet mit seinen Greifoberflächen 32 eine Neutralelektrode.

Am zweiten Pol der Konvertereinheit 14 ist das Schneidinstrument 10 angeschlossen, so dass bei Betätigen des entsprechenden Druckschalters eine elektrische Verbindung zwischen dem entsprechenden Pol der Konvertereinheit 14 und der Schneidelektrode 22 des Schneidinstrumentes 10 gegeben ist. Es kommt dann zu den durch Pfeile 60 schematisch dargestellten Stromfluss von der Schneidelektrode 22 zu den Greifoberflächen 32, wobei sich auf Grund der Feldstärkekonzentration die Schneidelektrode 22 als aktive Elektrode ausbildet und es zu einer Funkenentladung zwischen der Spitze der Schneidelektrode 22 und dem abzutrennenden Körpergewebe kommt.

Die Konvertereinheit 14 ist dabei die ganze Zeit an den symbolisch dargestellten Generator 1' angeschlossen, der eine Steuereinheit aufweist, die ein Abschalten des Stromes im Falle eines Kurzschlusses oder bei Überschreiten eines maximalen Impedanzgrenzwertes bewirkt.

Für den in Figur 8 dargestellten Anwendungsfall kann das verwendete Greifinstrument auch entsprechend der eingangs beschriebenen zweiten Ausführungsvariante als unipolares Greifinstrument ausgebildet sein, bei dem beide Greifoberflächen 32 mit einem gemeinsamen elektrischen Anschluss verbunden sind.

Ein Verfahren zur Anwendung der beschriebenen Medizingeräteanordnung besteht darin, dass zunächst die Anordnung, wie beispielhaft in Figur 8 dargestellt, zusammengestellt wird. Der Arzt greift dann als nächstes den zu entfernenden Gewebeteil mit den Greifinstrument 12. Da das Greifinstrument 12 als Gegenelektrode an dem zu entfernenden Gewebeteil ansetzt, kommt es zu einem Stromfluss im wesentlichen nur in dem zu entfernenden Gewebeteil, so dass potentielle Verbrennungen durch schlechten elektrischen Kontakt beispielsweise zwischen dem Greifinstrument 12 und dem zu entfernenden Gewebeteil unproblematisch sind.

Anschließend führt der Arzt das Schneidinstrument 10 und genauer gesagt die Schneidelektrode 22 in den Bereich des vorgesehenen Gewebeschnitts. Er kann durch Drücken des entsprechenden Druckschalters die elektrische Verbindung zwischen Schneidelektrode 22 und dem entsprechenden Pol der Konvertereinheit 14 herstellen, so dass es bei ausreichender Annäherung der Schneidelektrode 22 an das Körpergewebe zu einer Funkenstrecke zwischen Schneidelektrode 22 und Körpergewebe kommt, durch die das Körpergewebe getrennt wird. Der entsprechende Strom fließt zwischen der Schneidelektrode durch den abzutrennenden Körpergewebeteil zu den wegen ihrer größeren Oberfläche als Neutralelektrode wirkenden Greifoberflächen 32 des Greifinstrumentes. Während dieser Operation hält der Arzt den zu entfernenden Körpergewebeteil mit dem Greifinstrument 12 fest, um ihn nach vollständigem Abtrennen vom übrigen Körpergewebe mit Hilfe des Greifinstrumentes 12 entfernen zu können. Damit ist die Elektrotomie abgeschlossen.

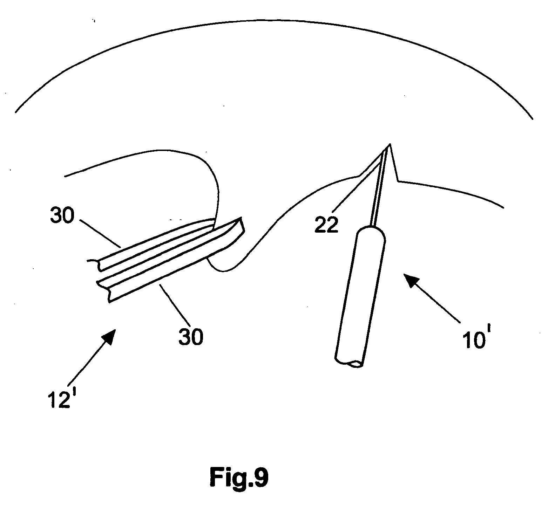

In Figur 9 ist dargestellt, wie die zuvor beschriebene Medizingeräteanordnung, beispielsweise für Einschnitte in ein Gaumensegel, verwendet werden kann. Dazu wird die Uvula mit dem Greifinstrument 12' in eine Richtung gezogen (in Figur 9 nach links) und gleichzeitig mit dem Schneidinstrument 10' ein Einschnitt ausgeführt. Auf Grund des Zuges mit dem Greifinstrument 12' öffnet sich der Einschnitt, wie in Figur 9 dargestellt.

Alternativ zu der in Figur 8 beispielhaft dargestellten Kürzung einer Uvula (Uvulapalatopharyngoplastik; UPPP) ist beispielsweise auch die Kürzung eines Gaumensegels ebenfalls zur Behandlung der Rhonchopathie möglich. Weitere Anwendungsfelder sind ebenfalls denkbar. Zu solchen weiteren Anwendungsfeldern zählen die Entfernung oder die Reduzierung der Tonsillen. Außerdem kann die Medizingeräteanordnung auch in der Dermatologie eingesetzt werden, beispielsweise zur Warzenbehandlung. Eine Warze kann beispielsweise mit einem Greifinstrument gegriffen werden und dann mit einem Schneidinstrument, beispielsweise mit einer Drahtschlaufe wie in Figur 1 dargestellt, ausgerüstet, vom übrigen Gewebe abschälend getrennt werden.

## Patentansprüche

1. Medizingeräteanordnung für das Schneiden von Körpergewebe mittels E-lektrotomie, mit
- einem Generator (1) für einen hochfrequenten Wechselstrom, der über einen Pol und einen Gegenpol abgegeben werden kann, wobei jeder Pol mit jeweils wenigstens einem Ausgangsanschluss (2) des Generators (1) verbunden ist und wobei der Generator zum Erzeugen einer niedrigen, insbesondere für den Zweck einer Gewebekoagulation ausgelegten Ausgangsspannung ausgebildet ist,
- einer Konvertereinheit (14),
- einem Greifinstrument (12) mit zwei Greifarmen (30), die jeweils wenigstens eine Greifoberfläche (32) aufweisen und derart mechanisch miteinander verbunden sind, dass die Greifoberflächen (32) aufeinander zu beweglich sind, und
- einem Schneidinstrument (10), welches einen Handgriff (26) und eine Schneidelektrode (22) aufweist,
wobei die Konvertereinheit (14) zwischen Generator und Greifinstrument (12) sowie Schneidinstrument (10) geschaltet und generatorseitig als separate Einheit an den mit Pol und Gegenpol des Generators (1) verbundenen Ausgangsanschluss (2) des Generators (1) angeschlossen und ausgangseitig mit dem Greifinstrument (12) einerseits und dem Schneidinstrument (10) andererseits derart verbunden ist, dass zu einem gegebenen Zeitpunkt beide Greifoberflächen (32) des Greifinstrumentes (12) ein gemeinsames erstes Potential und die Schneidelektrode (22) ein zweites Potential aufweisen, so dass im Betrieb der Medizingeräteanordnung ein hochfrequenter Wechselstrom zwischen dem Greifinstrument (12) und dem Schneidinstrument (10) fließt und **dadurch gekennzeichnet dass**, die Konvertereinheit einen Hochfrequenztransformator aufweist, bei dem das Verhältnis der Windungszahl (n₂) der Sekundärwicklung (44) zur Windungszahl (n₁) der Primärwicklung (42) n₂ > n₁ ist, so dass der Hochfrequenztransformator (40) im Betrieb eine niedrigere Ausgangsspannung des Generators in höhere Ausgangsspannungen hochtransformiert, die insbesondere das Zünden eines Funkens einer Schneidelektrode und damit das Durchtrennen von biologischem Gewebe ermöglichen,

2. Medizingeräteanordnung nach Anspruch 1, **dadurch gekennzeichnet, dass** die Konvertereinheit (14) jeweils einen Kondensator (50, 52) zwischen der Sekundärwicklung (44) und dem ersten Anschluss (46) einerseits und der Sekundärwicklung (44) und dem zweiten Anschluss (48) andrerseits aufweist.

3. Medizingeräteanordnung nach Anspruch 2, **dadurch gekennzeichnet, dass** die Kondensatoren (50, 52) zur Impedanzanpassung austauschbar angeordnet sind.

4. Medizingeräteanordnung nach einem der Ansprüche 1 bis 3, **dadurch gekennzeichnet, dass** der zweite Anschluss zwei parallelgeschaltete Anschlüsse (48) für das Greifinstrument aufweist.

5. Medizingeräteanordnung nach einem der Ansprüche 1 bis 4, **dadurch gekennzeichnet, dass** die Ausgangsspannung des Generators (1) 80 V ist und die Ausgangsspannung an der Konvertereinheit (14) 200 V.

6. Medizingeräteanordnung nach einem der Ansprüche 1 bis 5, **dadurch gekennzeichnet, dass** das Greifinstrument (12) ein bipolares Greifinstrument ist, bei dem die Greifoberflächen (32) jeweils wenigstens teilweise elektrisch leitend ausgebildet und jeweils einzeln über je einen, mit der jeweiligen Greifoberfläche verbundenen elektrischen Anschluss für eine elektrische Zuleitung (16) oder über einen gemeinsamen Anschluss für eine elektrische Zuleitung (16) elektrisch derart miteinander verbunden sind, dass beide Greifoberflächen elektrisch parallelgeschaltet sind.

7. Medizingeräteanordnung nach einem der Ansprüche 1 bis 6, **dadurch gekennzeichnet, dass** an die Konvertereinheit (14) einerseits das Schneidinstrument (10') und andererseits das Greifinstrument (12) über entsprechende elektrische Zuleitungen (16) angeschlossen sind und die Konvertereinheit (14) eingangsseitig ein Kabel (18) mit daran angeschlossenem Anschlussstecker (20) für den Anschluss der Konvertereinheit (14) an den Generator (1) aufweist.

8. Medizingeräteanordnung nach einem der Ansprüche 1 bis 7, wobei das Greifinstrument (12) wahlweise einen geöffneten oder einen geschlossenen Zustand annehmen kann, wobei die jede Greifoberfläche (32) wenigstens teilweise elektrisch leitend ausgebildet, gegenüber der jeweils anderen Greifoberfläche (32) elektrisch isoliert und jeweils einzeln mit einem Anschluss für je eine elektrische Zuleitung elektrisch verbunden ist, **dadurch gekennzeichnet, dass** die beiden elektrischen Zuleitungen über eine Verzweigung mit einer elektrischen Zuleitung zu einem Schneidinstrument fest verbunden und die beiden Zuleitungen über die Verzweigung mit einer gemeinsamen Zuleitung für den Anschluss an einen Generator oder eine Konvertereinheit verbunden sind.

9. Medizingeräteanordnung nach einem der Ansprüche 1 bis 7, wobei das Greifinstrument (12) wahlweise einen geöffneten oder einen geschlossenen Zustand annehmen kann, wobei die Greifoberfläche (32) wenigstens teilweise elektrisch leitend ausgebildet und mit einem gemeinsamen Anschluss für eine elektrische Zuleitung elektrisch verbunden sind, **dadurch gekennzeichnet, dass** die elektrische Zuleitung über eine Verzweigung mit einer elektrischen Zuleitung zu einem Schneidinstrument fest verbunden und die beiden Zuleitungen über die Verzweigung mit einer gemeinsamen Zuleitung für den Anschluss an einen Generator oder eine Konvertereinheit verbunden sind.

10. Medizingeräteanordnung nach Anspruch 8 oder 9, **dadurch gekennzeichnet, dass** die Greifoberfläche (32) eine Oberflächenstruktur mit Erhebungen aufweist, die ein sicheres Greifen von Körperteilen erlaubt.

11. Medizingeräteanordnung nach Anspruch 10, **dadurch gekennzeichnet, dass** die Erhebungen die Gestalt von Fasszähnen (34) haben.

12. Medizingeräteanordnung nach einem der Ansprüche 8 bis 11, **dadurch gekennzeichnet, dass** eine jeweilige Greifoberfläche (32) Bestandteil eines jeweils austauschbar an dem Greifinstrument (12) zu befestigenden Greifansatzes ist.

13. Medizingeräteanordnung nach Anspruch 12, **dadurch gekennzeichnet, dass** ein jeweiliger Greifansatz auf ein jeweiliges distales Ende eines Greifarmes (30) aufzuklemmen ist.

14. Medizingeräteanordnung nach Anspruch 12 oder 13, **dadurch gekennzeichnet, dass** der Greifansatz aus Edelstahl gefertigt ist.

15. Medizingeräteanordnung nach einem der Ansprüche 8 bis 14, **dadurch gekennzeichnet, dass** das Greifinstrument (12) nach Art einer Pinzette gestaltet ist.

16. Medizingeräteanordnung nach einem der Ansprüche 8 bis 14, **dadurch gekennzeichnet, dass** das Greifinstrument (12) nach Art einer Fasszange gestaltet ist.

17. Medizingeräteanordnung nach einem der Ansprüche 8 bis 16, **dadurch gekennzeichnet, dass** das Greifinstrument (12) ohne äußere Krafteinwirkung selbständig den geöffneten Zustand annimmt.

18. Medizingeräteanordnung nach einem der Ansprüche 8 bis 16, **dadurch gekennzeichnet, dass** das Greifinstrument (12) nach Einstellen des geschlossenen Zustands diesen beibehält oder im geschlossenen Zustand zu verriegeln ist.

19. Medizingeräteanordnung nach einem der Ansprüche 8 bis 16, **dadurch gekennzeichnet, dass** das Greifinstrument (12) im geschlossenen Zustand federnd vorgespannt und gegen eine Federkraft zu öffnen ist.

20. Medizingeräteanordnung nach einem der Ansprüche 1 bis 7, bei dem der Handgriff (26) des Schneidinstrumentes eine Greifoberfläche (32) aufweist, die gegenüber der Schneidelektrode (22) isoliert ist und dass der Handgriff (26) einen Anschluss für eine elektrische Zuleitung aufweist, der elektrisch mit der Schneidelektrode (22) verbunden ist, wobei die Schneidelektrode (22) an einem distalen Ende eines Elektrodenschaftes (24) angeordnet und über den Elektrodenschaft (24) mit dem Handgriff (26) verbunden ist, **dadurch gekennzeichnet, dass** die elektrische Zuleitung über eine Verzweigung mit einer elektrischen Zuleitung zu dem Greifinstrument fest verbunden und die beiden Zuleitungen über die Verzweigung mit einer gemeinsamen Zuleitung für den Anschluss an einen Generator oder eine Konvertereinheit verbunden sind.

21. Medizingeräteanordnung nach Anspruch 20, **dadurch gekennzeichnet, dass** die Schneidelektrode (22) angespitzt ist.

22. Medizingeräteanordnung nach Anspruch 20 oder 21, **dadurch gekennzeichnet, dass** die Schneidelektrode als Drahtschlaufe ausgebildet ist.

23. Medizingeräteanordnung nach einem der Ansprüche 20 bis 22, **dadurch gekennzeichnet, dass** die Schneidelektrode oder die Schneidelektrode samt Elektrodenschaft auswechselbar mit dem übrigen Schneidinstrument, insbesondere dem Handgriff, verbunden ist.

24. Medizingeräteanordnung nach einem der Ansprüche 20 bis 23, **gekennzeichnet durch** einen an dem Handgriff angeordneten, mit einem Finger zu betätigenden Druckschalter, mit dem eine elektrische Verbindung zu der Schneidelektrode wahlweise herzustellen oder zu unterbrechen ist.

## Claims

1. Medical device assembly for cutting body tissue by means of electrotomy, comprising
- a generator (1) for a high frequency alternating current, which can be delivered by way of a pole and an opposite pole, wherein each pole is connected to at least one respective output terminal (2) of the generator (1), and wherein the generator is designed to generate a low output voltage intended in particular for the purpose of tissue coagulation,
- a converter unit (14),
- a gripping instrument (12) with two gripping arms (30), which each have at least one respective gripping surface (32) and which are joined together mechanically in such a way that the gripping surfaces (32) can be moved towards each other, and
- a cutting instrument (10) which has a handle (26) and a cutting electrode (22),
wherein the converter unit (14) is connected between the generator, the gripping instrument (12) and the cutting instrument (10) and is connected on the generator side as a separate unit to the output terminal (2) of the generator (1) which is connected to the pole and opposite pole of the generator (1), and on the output side is connected to the gripping instrument (12) on the one hand and the cutting instrument (10) on the other hand, in such a way that at a given moment both gripping surfaces (32) of the gripping instrument (12) have a common first potential and the cutting electrode (22) has a second potential, so that during the operation of the medical device assembly a high frequency alternating current flows between the gripping instrument (12) and the cutting instrument (10), **characterised in that** the converter unit has a high frequency transformer, in which the ratio of the number of turns per centimetre (n₂) of the secondary winding (44) to the number of turns per centimetre (n₁) of the primary winding (42) is n₂ > n₁, so that the high frequency transformer (40) during operation transforms a lower output voltage of the generator up to higher output voltages, which in particular enable the ignition of a spark of a cutting electrode and thus the cutting of biological tissue.

2. Medical device assembly according to claim 1, **characterised in that** the converter unit (14) comprises respectively a capacitor (50, 52) between the secondary winding (44) and the first terminal (46) on the one hand and the secondary winding (44) and the second terminal (48) on the other hand.

3. Medical device assembly according to claim 2, **characterised in that** the capacitors (50, 52) for adjusting impedance are arranged to be replaceable.

4. Medical device assembly according to one of claims 1 to 3, **characterised in that** the second terminal comprises two parallel connected terminals (48) for the gripping instrument.

5. Medical device assembly according to one of claims 1 to 4, **characterised in that** the output voltage of the generator (1) is 80 V and the output voltage at the converter unit (14) is 200 V.

6. Medical device assembly according to one of claims 1 to 5, **characterised in that** the gripping instrument (12) is a bipolar gripping instrument, in which the gripping surfaces (32) are designed to be at least partly electrically conductive and are connected electrically to one another individually via an electrical terminal connected to the respective gripping surface for an electric supply cable (16) or via a joint terminal for an electrical supply cable (16), in such a way that both gripping surfaces are connected in parallel electrically.

7. Medical device assembly according to one of claims 1 to 6, **characterised in that** on the one hand the cutting instrument (10') and on the other hand the griping instrument (12) are connected to the converter unit (14) via corresponding electrical supply cables (16), and the converter unit (14) on the input side has a cable (18) with connected plugs (20) for the connection of the converter unit (14) to the generator (1).

8. Medical device assembly according to one of claims 1 to 7, wherein the gripping instrument (12) optionally can adopt an open or closed position, whereby each gripping surface (32) is designed to at least partially conduct electricity, relative to which the respective other gripping surface (32) is electrically insulated and is connected electrically individually to the terminal for an electrical supply cable, **characterised in that** the two electric supply cables are securely connected via a branching with an electrical supply cable to a cutting instrument, and the two supply cables are connected via the branching with a common supply cable for connection to a generator or a converter unit.

9. Medical device assembly according to one of claims 1 to 7, wherein the gripping instrument (12) optionally can adopt an open or closed position, whereby the gripping surface (32) is designed to at least partially conduct electricity and is connected electrically individually with a joint terminal for an electrical supply cable, **characterised in that** the electrical supply cable is securely connected via a branching with an electrical supply cable to a cutting instrument and the two supply cables are connected via the branching with a joint supply cable for the terminal to a generator or a converter unit.

10. Medical device assembly according to claim 8 or 9, **characterised in that** the gripping surface (32) has a surface structure with raised portions, which enables the secure gripping of body parts.

11. Medical device assembly according to claim 10, **characterised in that** the raised portions are in the form of grabbing teeth (34).

12. Medical device assembly according to one of claims 8 to 11, **characterised in that** a respective gripping surface (32) is a component of a respective gripping attachment which is to be secured replaceably onto the gripping instrument (12).

13. Medical device assembly according to claim 12, **characterised in that** a respective gripping attachment is to be clamped onto a respective distal end of a gripping arm (30).

14. Medical device assembly according to claim 12 or 13, **characterised in that** the gripping attachment is made from high-quality steel.

15. Medical device assembly according to one of claims 8 to 14, **characterised in that** the gripping instrument (12) is designed in the form of a pair of tweezers.

16. Medical device assembly according to one of claims 8 to 14, **characterised in that** the gripping instrument (12) is designed in the form of holding tongs.

17. Medical device assembly according to one of claims 8 to 16, **characterised in that** the gripping instrument (12) automatically assumes an open position without an external force acting thereon.

18. Medical device assembly according to one of claims 8 to 16, **characterised in that** after setting the closed position the gripping instrument (12) maintains this position or is locked in the closed position.

19. Medical device assembly according to one of claims 8 to 16, **characterised in that** in the closed condition the gripping instrument (12) is resiliently tensioned and opens against a resilient force.

20. Medical device assembly according to one of claims 1 to 7 in which the handle (26) of the cutting instrument has a gripping surface (32), which is insulated from the cutting electrode (22), and in that the handle (26) has a terminal for an electrical supply cable, which is electrically connected to the cutting electrode (22), wherein the cutting electrode (22) is arranged at a distal end of an electrode shaft (24) and is connected to the handle (26) via the electrode shaft (24), **characterised in that** the electrical supply cable is securely connected via a branching with an electrical supply cable to the gripping instrument and the two supply cables are connected via the branching to a common supply cable for connection to a generator or a converter unit.

21. Medical device assembly according to claim 20, **characterised in that** the cutting electrode (22) is pointed.

22. Medical device assembly according to claim 20 or 21, **characterised in that** the cutting electrode is in the form of a wire loop.

23. Medical device assembly according to one of claims 20 to 22, **characterised in that** the cutting electrode or the cutting electrode together with the electrode shaft is replaceably connected to the rest of the cutting instrument, in particular the handle.

24. Medical device assembly according to one of claims 20 to 23, **characterised by** a press switch which is arranged on the handle and which can be activated by a finger, and with which an electrical connection to the cutting electrode can be made or interrupted selectively.

## Revendications

1. Appareil médical pour la coupe de tissus corporels au moyen de l'électrotomie, comportant
- un générateur (1) pour un courant alternatif haute fréquence qui peut être délivré par l'intermédiaire d'un pôle et d'un pôle opposé, chacun des pôles étant respectivement connecté à au moins une borne de sortie (2) du générateur (1) et le générateur étant réalisé pour produire une basse tension de sortie, appliquée en particulier à des fins de coagulation des tissus,
- un bloc convertisseur (14),
- un instrument de préhension (12) comportant deux bras de préhension (30) qui présentent chacun au moins une surface de préhension (32) et sont reliés mécaniquement l'un à l'autre de telle manière que les surfaces de préhension (32) peuvent se déplacer l'une vers l'autre, et
- un instrument de coupe (10) qui présente une poignée (26) et une électrode de coupe (22),
le bloc convertisseur (14) étant monté entre le générateur et l'instrument de préhension (12) ainsi que l'instrument de coupe (10) et étant raccordé côté générateur en tant qu'unité séparée à la borne de sortie (2) du générateur (1) connectée au pôle et au pôle opposé du générateur (1) et étant connecté côté sortie à l'instrument de préhension (12) d'une part et à l'instrument de coupe (10) d'autre part de telle manière qu'à un moment donné, les deux surfaces de préhension (32) de l'instrument de préhension (12) présentent un premier potentiel commun et l'électrode de coupe (22) présente un deuxième potentiel, de sorte que lors du fonctionnement de l'appareil médical, un courant alternatif haute fréquence circule entre l'instrument de préhension (12) et l'instrument de coupe (10), **caractérisé en ce que** le bloc convertisseur présente un transformateur haute fréquence dans lequel le rapport entre le nombre de spires (n₂) de l'enroulement secondaire (44) et le nombre de spires (n₁) de l'enroulement primaire (42) est n₂ > n₁, de sorte qu'en fonctionnement, le transformateur haute fréquence (40) transforme une basse tension de sortie du générateur en hautes tensions de sortie qui permettent en particulier l'allumage d'une étincelle d'une électrode de coupe et ainsi le sectionnement de tissus biologiques.

2. Appareil médical selon la revendication 1, **caractérisé en ce que** le bloc convertisseur (14) présente un condensateur (50, 52) placé respectivement entre l'enroulement secondaire (44) et la première borne (46) d'une part et entre l'enroulement secondaire (44) et la deuxième borne (48) d'autre part.

3. Appareil médical selon la revendication 2, **caractérisé en ce que** les condensateurs (50, 52) sont disposés pour être remplaçables à des fins d'adaptation à l'impédance.

4. Appareil médical selon une des revendications 1 à 3, **caractérisé en ce que** la deuxième borne présente deux bornes montées en parallèle (48) pour l'instrument de préhension.

5. Appareil médical selon une des revendications 1 à 4, **caractérisé en ce que** la tension de sortie du générateur (1) est de 80 V et la tension de sortie au niveau du bloc convertisseur (14) est de 200 V.

6. Appareil médical selon une des revendications 1 à 5, **caractérisé en ce que** l'instrument de préhension (12) est un instrument bipolaire, sur lequel les surfaces de préhension (32) sont chacune réalisées pour être au moins partiellement électroconductrices, et sont chacune connectées l'une à l'autre individuellement par l'intermédiaire d'une borne électrique destinée à une ligne d'alimentation électrique (16) et connectée à la surface de préhension concernée, ou par l'intermédiaire d'une borne commune destinée à une ligne d'alimentation électrique (16) de telle manière que les deux surfaces de préhension sont montées électriquement en parallèle.

7. Appareil médical selon une des revendications 1 à 6, **caractérisé en ce que** d'une part l'instrument de coupe (10) et d'autre part l'instrument de préhension (12) sont raccordés au bloc convertisseur (14) par l'intermédiaire des lignes d'alimentation électriques (16) correspondantes et **en ce que** le bloc convertisseur (14) présente côté entrée un câble (18) auquel est raccordé une prise mâle (20) pour le raccordement du bloc convertisseur (14) au générateur (1).

8. Appareil médical selon une des revendications 1 à 7, l'instrument de préhension (12) pouvant adopter au choix un état ouvert ou un état fermé, chaque surface de préhension (32) étant réalisée pour être au moins partiellement électroconductrice, chacune étant isolée électriquement de l'autre surface de préhension (32) et chacune étant connectée individuellement à une borne destinée à une ligne d'alimentation électrique, **caractérisé en ce que** les deux lignes d'alimentation électriques sont connectées fixement par l'intermédiaire d'une jonction à une ligne d'alimentation électrique alimentant un instrument de coupe, et **en ce que** les deux lignes d'alimentation sont connectées par l'intermédiaire de la jonction à une ligne d'alimentation commune pour le raccordement à un générateur ou un ensemble convertisseur.

9. Appareil médical selon une des revendications 1 à 7, l'instrument de préhension (12) pouvant adopter au choix un état ouvert ou un état fermé, les surfaces de préhension (32) étant réalisées pour être au moins partiellement électroconductrices, et étant connectées à une borne commune destinée à une ligne d'alimentation électrique, **caractérisé en ce que** la ligne d'alimentation électrique est connectée fixement par l'intermédiaire d'une jonction à une ligne d'alimentation électrique alimentant un instrument de coupe, et **en ce que** les deux lignes sont connectées par l'intermédiaire de la jonction à une ligne d'alimentation commune pour le raccordement à un générateur ou à un bloc convertisseur.

10. Appareil médical selon la revendication 8 ou 9, **caractérisé en ce que** la surface de préhension (32) présente une structure de surface comportant des saillies qui permettent une prise sûre de fragments de corps.

11. Appareil médical selon la revendication 10, **caractérisé en ce que** les saillies ont la forme de dents de prise (34).

12. Appareil médical selon une des revendications 8 à 11, **caractérisé en ce que** chaque surface de préhension (32) est partie intégrante d'un insert de préhension remplaçable destiné à être fixé à l'instrument de préhension (12).

13. Appareil médical selon la revendication 12, **caractérisé en ce que** chaque insert de préhension est destiné à être fixé sur une extrémité distale d'un bras de préhension (30).

14. Appareil médical selon la revendication 12 ou 13, **caractérisé en ce que** l'insert de préhension est constitué d'acier inoxydable.

15. Appareil médical selon une des revendications 8 à 14, **caractérisé en ce que** l'instrument de préhension (12) est réalisé à la manière d'une pincette.

16. Appareil médical selon une des revendications 8 à 14, **caractérisé en ce que** l'instrument de préhension (12) est réalisé à la manière d'une pince de prise.

17. Appareil médical selon une des revendications 8 à 16, **caractérisé en ce que** l'instrument de préhension (12) adopte automatiquement l'état ouvert sans que soit exercée une force extérieure.

18. Appareil médical selon une des revendications 8 à 16, **caractérisé en ce que** l'instrument de préhension (12), une fois mis à l'état fermé, conserve cet état ou doit être verrouillé à l'état fermé.

19. Appareil médical selon une des revendications 8 à 16, **caractérisé en ce qu'**à l'état fermé, l'instrument de préhension (12) est précontraint par ressort et doit être ouvert à l'encontre d'une force de ressort.

20. Appareil médical selon une des revendications 1 à 7, sur lequel la poignée (26) de l'instrument de coupe présente une surface de préhension (32) qui est isolée par rapport à l'électrode de coupe (22) et en ce que la poignée (26) présente une borne destinée à une ligne d'alimentation électrique qui est connectée électriquement à l'électrode de coupe (22), l'électrode de coupe (22) étant disposée à une extrémité distale d'une tige d'électrode (24) et étant connectée à la poignée (26) par l'intermédiaire de la tige d'électrode (24), **caractérisé en ce que** la ligne d'alimentation électrique est connectée fixement par l'intermédiaire d'une jonction à une ligne d'alimentation électrique alimentant l'instrument de préhension et **en ce que** les deux lignes d'alimentation sont connectées par l'intermédiaire de la jonction à une ligne d'alimentation commune pour le raccordement à un générateur ou à un ensemble convertisseur.

21. Appareil médical selon la revendication 20, **caractérisé en ce que** l'électrode de coupe (22) est pointue.

22. Appareil médical selon la revendication 20 ou 21, **caractérisé en ce que** l'électrode de coupe est réalisée sous la forme d'une boucle de fil.

23. Appareil médical selon une des revendications 20 à 22, **caractérisé en ce que** l'électrode de coupe ou l'électrode de coupe et la tige d'électrode sont connectées de manière interchangeable à l'autre instrument de coupe, en particulier la poignée.

24. Appareil médical selon une des revendications 20 à 23, **caractérisé par** un interrupteur à poussoir disposé sur la poignée et actionné par un doigt, et avec lequel une connexion électrique alimentant l'électrode de coupe peut être au choix établie ou interrompue.
